(19) Europäisches Patentamt — European Patent Office — Office européen des brevets

(11) **EP 3 066 459 B1**

(12) **EUROPÄISCHE PATENTSCHRIFT**

(45) Veröffentlichungstag und Bekanntmachung des Hinweises auf die Patenterteilung:
**28.11.2018  Patentblatt 2018/48**

(21) Anmeldenummer: **14809750.4**

(22) Anmeldetag: **15.10.2014**

(51) Int Cl.:
**G01N 27/414** $^{(2006.01)}$     **G01N 33/543** $^{(2006.01)}$

(86) Internationale Anmeldenummer:
**PCT/DE2014/000515**

(87) Internationale Veröffentlichungsnummer:
**WO 2015/067231 (14.05.2015 Gazette 2015/19)**

(54) **VORRICHTUNG UND VERFAHREN ZUR MESSUNG KLEINER SPANNUNGEN UND POTENTIALE AN EINER BIOLOGISCHEN, CHEMISCHEN ODER ANDEREN PROBE**

APPARATUS AND METHOD FOR MEASURING SMALL VOLTAGES AND POTENTIALS AT A BIOLOGICAL, CHEMICAL OR OTHER SAMPLE

DISPOSITIF ET PROCÉDÉ DE MESURE DE TENSIONS ET POTENTIELS ÉLECTRIQUES FAIBLES SUR UN ÉCHANTILLON CHIMIQUE OU BIOLOGIQUE OU D'AUTRES ÉCHANTILLONS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.11.2013   DE 102013018850**

(43) Veröffentlichungstag der Anmeldung:
**14.09.2016   Patentblatt 2016/37**

(73) Patentinhaber: **Forschungszentrum Jülich GmbH 52425 Jülich (DE)**

(72) Erfinder:
- **VITUSEVICH, Svetlana**
  **52353 Düren (DE)**
- **LI, Jing**
  **52428 Jülich (DE)**
- **PUD, Sergii**
  **2611 CD Delft (NL)**

(56) Entgegenhaltungen:
WO-A2-2013/025429     DE-A1-102010 062 224
US-A1- 2006 231 754

- FEI LIU ET AL: "Determination of the Small Band Gap of Carbon Nanotubes Using the Ambipolar Random Telegraph Signal", NANO LETTERS, Bd. 5, Nr. 7, 1. Juli 2005 (2005-07-01), Seiten 1333-1336, XP055170634, ISSN: 1530-6984, DOI: 10.1021/nl050578c & FEI LIU ET AL: "Giant random telegraph signals in the carbon nanotubes as a single defect probe", APPLIED PHYSICS LETTERS, AMERICAN INSTITUTE OF PHYSICS, US, Bd. 86, Nr. 16, 11. April 2005 (2005-04-11), Seiten 163102-163102, XP012065133, ISSN: 0003-6951, DOI: 10.1063/1.1901822
- NENG-PING WANG ET AL: "Effects of defects near source or drain contacts of carbon nanotube transistors", EUROPHYSICS LETTERS: A LETTERS JOURNAL EXPLORING THE FRONTIERS OF PHYSICS, INSTITUTE OF PHYSICS PUBLISHING, BRISTOL, FR, Bd. 100, Nr. 4, 3. Dezember 2012 (2012-12-03), Seite 47009, XP020231212, ISSN: 0295-5075, DOI: 10.1209/0295-5075/100/47009
- CLEMENT N ET AL: "A silicon nanowire ion-sensitive field-effect transistor with elementary charge sensitivity", APPLIED PHYSICS LETTERS, AMERICAN INSTITUTE OF PHYSICS, US, Bd. 98, Nr. 1, 6. Januar 2011 (2011-01-06) , Seiten 14104-14104, XP012139118, ISSN: 0003-6951, DOI: 10.1063/1.3535958

EP 3 066 459 B1

• F. LIU ET AL: "Study of Random Telegraph Signals in Single-Walled Carbon Nanotube Field Effect Transistors", IEEE TRANSACTIONS ON NANOTECHNOLOGY, Bd. 5, Nr. 5, 1. September 2006 (2006-09-01), Seiten 441-445, XP055172397, ISSN: 1536-125X, DOI: 10.1109/TNANO.2006.880906

**Beschreibung**

Die Erfindung betrifft eine Vorrichtung und Verfahren zur Messung kleiner Spannungen und Potentiale an biologischen, chemischen und anderen Proben.

Stand der Technik

Viele Eigenschaften biologischer Proben hängen mit der Aufnahme, Abgabe oder räumlichen Umorganisation elektrischer Ladungen zusammen und können daher indirekt und nichtinvasiv über Veränderungen von Potentialen gemessen werden, die diese Ladungen bei Annäherung an eine Messvorrichtung verursachen. Derartige gattungsgemäße Messvorrichtungen enthalten häufig einen Feldeffekttransistor, dessen Gate mit der Probe in Kontakt steht. Die Probe ändert das Potential am Gate und moduliert damit den bei vorgegebener Drain-Source-Spannung durch den Transistor getriebenen Strom. Nachteilig ist das Signal derartiger Messungen zur Untersuchung kleiner Spannungen und Potentiale an biologischen, chemischen und anderen Proben zu gering.

Aus (S. Sorgenfrei, C. Chiu, M. Johnston, C. Nuckolls, K. L. Shepard, "Debye Screening in Single-Molecule Carbon Nanotube Field-Effect Sensors", Nano Letters 2011 (11), 3739-3743 (2011)) ist eine Messvorrichtung bekannt, die ein Kohlenstoff-Nanoröhrchen als Feldeffekttransistor verwendet. Das Nanoröhrchen ist zur Anlagerung eines Biomoleküls mit einer Fehlstelle funktionalisiert. Das Binden eines Biomoleküls an diese Fehlstelle sowie das Entkoppeln des Biomoleküls von dieser Fehlstelle produzieren im durch das Nanoröhrchen getriebenen Strom ein Zwei-Niveau-Telegrafenrauschen, mit dem die Kinetik des Bindens und Entkoppelns studiert werden kann.

Aus Fei Liu et al.: "Determination of the Small Band Gap of Garbon Nanotubes Using the Ambipolar Random Telegraph Signal", NANO LETTERS, Bd. 5, Nr. 7, 1. Juli 2005 (2005-07-01), Seiten 1333-1336 sind die elektrischen Eigenschaften eines Carbon-Nanotube-Feldeffekttransistors (CNT-FET) bekannt. Der CNT-FET ist derart empfindlich, dass einzelne Ladungen detektiert werden können. In diesem Zusammenhang verweist die Druckschrift auf ein weiteres Dokument derselben Autoren (Fei Liu et al.: "Giant random telegraph signals in the carbon nanotubes as a single defect probe", APPLIED PHYSICS LETTERS, AMERICAN INSTITUTE OF PHYSICS, US, Bd. 86, Nr. 16, 11. April 2005 (2005-04-11), Seiten 163102-163102), in der der Aufbau des verwendeten Transistors beschrieben wird. Auf einem Si-Substrat ist ein $SiO_2$ umfassendes Gate-Dielektrikum mit unterschiedlichen Defekten (A und B) angeordnet. Auf diesen werden Nanotubes mit einem Durchmesser von 1 bis 3 nm und einer Länge von ca. 4 $\mu$m generiert. Auf den Nanotubes sind die Elektroden für Source und Drain und die entsprechenden Kontakte angeordnet. Beim Anlegen einer Spannung an Source und Drain können durch weiteres Anlegen unterschiedlicher Gatespannungen RTS-Signale erhalten werden, welche durch das Einfangen von Ladungen/Löchern aus der Nanotube in die Defekte und das Abgeben der Löcher/Ladungen aus den Defekten zurück in die Nanotube entstehen. Aus dem RTS-Signalen können somit Rückschlüsse auf die Defektstruktur innerhalb des Gate-Dielektrikums erhalten werden.

Ferner ist aus Neng-Ping Wang et al.: "Effects of defects near source or drain contacts of carbon nanotube transistors", EUROPHYSICS LEITERS: A LEDERS JOURNAL EXPLORING THE FRONTIERS OF PHYSICS, INSTITUTE OF PHYSICS PUBLISHING, BRISTOL, FR, Bd. 100, Nr. 4, 3. Dezember 2012 (2012-12-03), Seite 47009, ein auf Carbon-Nanotubes (CNT) basierender Feldeffekttransistor bekannt, bei dem ebenfalls das TRS-Signal untersucht wurde. Der CNT-FET umfasst Source, Drain, Gate und ein Gateoxid, die für elektrische Messungen kontaktierbar sind. Das Gateoxid und/oder die Grenzfläche zwischen Gateoxid und der Nanotube weist dabei wenigstens einen Defekt auf, der einen Ladungsträger aus der CNT einzufangen und wieder abzugeben vermag. Auch hier kann aus dem RTS-Signal auf die Defektstruktur des Gateoxids geschlossen werden, was für die Herstellung von CNT-FET wichtig ist.

Auch aus US 2006/231754 A1 ist ein CNT-FET bekannt, der allerdings als Mikroskop-Sonde eingesetzt wird, um die Defektstruktur einer Probe zu untersuchen. Dabei umfasst die Mikroskop-Sonde selbst einen FET-Aufbau umfassend ein defektfreies Gate-Dielektrikum mit Source, Drain und einer dazwischen angeordneten Nanotube auf einer ersten Seite, sowie einem Gate auf der Rückseite des Dielektrikums. Die Mikroskop-Sonde mit der herausstehenden Nanotube kann nun in unmittelbare Nähe zu einer Probe umfassend ein Defekt aufweisendes Dielektrikum gebracht werden. Beim Anlegen einer Spannung an Source und Drain können durch weiteres Anlegen unterschiedlicher Gatespannungen RTS-Signale erhalten werden, welche durch das Einfangen von Ladungen/Löchern aus der Nanotube in die Defekte der zu untersuchenden Probe und das Abgeben der Löcher/Ladungen aus den Defekten der zu untersuchenden Probe zurück in die Nanotube entstehen. Aus den RTS-Signalen können somit Rückschlüsse auf die Defektstruktur innerhalb der zu untersuchenden Probe erhalten werden.

Aufgabe und Lösung

Es ist die Aufgabe der Erfindung, über die Kinetik des Bindens und Entkoppelns an eine Fehlstelle hinaus die Vermessung weiterer Eigenschaften von Proben, die Potentialänderungen zu bewirken vermögen, quantitativ mit einer besseren Genauigkeit zu ermöglichen als nach dem bisherigen Stand der Technik.

Diese Aufgabe wird erfindungsgemäß gelöst durch eine Vorrichtung gemäß Hauptanspruch und durch ein Verfahren gemäß Nebenanspruch. Weitere vorteilhafte Ausgestaltungen ergeben sich aus den darauf rückbezogenen Unteransprüchen.

Gegenstand der Erfindung

Im Rahmen der Erfindung wurde eine Vorrichtung zur Messung kleiner Spannungen und Potentiale oder anderer elektrischer Größen einer Probe entwickelt. Diese Vorrichtung wird durch den unabhängigen Anspruch 1 definiert.

Indem eine derartige Anlagerstelle permanent statistisch Ladungsträger mit dem Kanal austauscht, überlagert sie den durch den Kanal und damit durch den Transistor fließenden Strom mit statistischen, stufenartigen Fluktuationen zwischen zwei Niveaus. Diese Niveaus entsprechen den beiden möglichen Zuständen, dass die Anlagerstelle mit einem Ladungsträger entweder besetzt oder nicht besetzt ist. Derartige Fluktuationen sind in der Halbleiterelektronik normalerweise unerwünscht, so dass Anstrengungen unternommen werden, um sie zu minimieren. Da der Zeitverlauf der Fluktuationen dem eines Morsesignals auf einer Telegrafenleitung ähnelt, hat sich für sie die Bezeichnung "Telegraphenrauschen" (random telegraph noise) eingebürgert.

Es wurde erkannt, dass die Wahrscheinlichkeiten für den Austausch von Ladungsträgern sehr empfindlich davon abhängen, wie das Potential am Ort der Anlagerstelle in Relation zum Fermi-Niveau steht. Bereits eine geringe Potentialänderung durch Annäherung eines Biomoleküls ändert diese Wahrscheinlichkeiten drastisch. Dies manifestiert sich in charakteristischen Kenngrößen der Fluktuationen, insbesondere in den Zeitkonstanten für das Einfangen und/oder für das Abgeben von Ladungsträgern durch die Anlagerstelle. Diese Zeitkonstanten entscheiden jeweils über die Dauer, für die jedes der beiden Stromniveaus vorliegt. Ein deutliches Signal ist bereits messbar, bevor das Biomolekül sich an das Gate bindet oder es auch nur berührt. Das Gate ist hierbei im Allgemeinen nicht metallisiert. Die Probe wird direkt mit dem Gate-Dielektrikum in Kontakt gebracht, das optional an einigen Stellen mit zusätzlichen Molekülen zur Bindung interessierender Probenmoleküle funktionalisiert sein kann.

Das durch die Fluktuationen bewirkte Nutzsignal, das aus plötzlichen stufenartigen Übergängen zwischen diskreten Stromniveaus besteht, wobei die Zeitpunkte dieser Übergänge jeweils statistisch verteilt sind, wird im Folgenden als Telegrafen-Modulationssignal bezeichnet.

Im Gegensatz zum bisherigen Stand der Technik wird das Telegrafen -Modulationssignal nicht an dem Ort erzeugt, wo die Probe mit der Messvorrichtung wechselwirkt, sondern im Inneren des Gate-Dielektrikums. Die Anlagerstelle kommt mit der Probe nicht unmittelbar in Berührung. Wenn sich die Charakteristik des Telegrafen-Modulationssignals ändert, ist dies daher nur durch die von der Probe bewirkte Potentialänderung bedingt und nicht durch sonstige Eigenschaften der Probe beeinflusst. Daher sind aufeinander folgende Messungen an verschiedenen Proben quantitativ miteinander vergleichbar.

Ursache für das im Vergleich zum Stand der Technik stärkere Signal ist zum Einen, dass die beiden Niveaus des Telegrafen-Modulationssignals durch eine größere Energielücke voneinander getrennt sind. Nach dem Stand der Technik unterscheiden sich die beiden Zustände nur um die Bindungsenergie des Biomoleküls an die Fehlstelle im Nanoröhrchen. Zum Austausch einer Ladung zwischen der erfindungsgemäß genutzten Fehlstelle im Gate-Dielektrikum und dem Kanal ist ein deutlich höherer Energiebetrag (Coulomb-Energie) notwendig. Zum Anderen greift der Unterschied zwischen diesen beiden Niveaus auch stärker auf den Strom durch den Transistor durch, indem die Anlagerstelle nahe am Kanal sitzt. Die Anlagerung eines Biomoleküls am Nanoröhrchen gemäß Stand der Technik wird dagegen lediglich über Bildladungseffekte an den Kanal vermittelt und greift daher deutlich schwächer auf den Strom durch.

Die Wirkung der Anlagerstelle kann in verschiedener Weise maßgeschneidert werden, um die Vorrichtung für bestimmte Messbereiche des Potentials besonders sensitiv zu machen. Hierfür können beispielsweise Abschirmeffekte innerhalb des Materials des Gate-Dielektrikums genutzt werden. Der Energiebetrag, der für den Austausch einer Ladung zwischen der Anlagerstelle und dem Kanal notwendig ist, sowie der Wirkungsquerschnitt für diesen Ladungsaustausch lassen sich unter anderem durch die Position der Anlagerstelle innerhalb des Gate-Dielektrikums vorgeben. Zugleich lässt sich das Fermi-Niveau über die Biasspannung am Gate in weiten Grenzen verschieben. Das Gate-Dielektrikum kann vorteilhaft mehrere Anlagerstellen enthalten, die sich in den Energiebeträgen für den Ladungsaustausch unterscheiden, so dass Messungen in mehreren Messbereichen simultan durchgeführt werden können. Damit die Signalbeiträge noch sicher voneinander unterschieden werden können, sollten jedoch nicht mehr als fünf Anlagerstellen gleichzeitig aktiv sein.

In einer besonders vorteilhaften Ausgestaltung der Erfindung ist die Anlagerstelle ein lokaler Defekt im Gate-Dielektrikum. Dieser kann vorteilhaft insbesondere

- durch eine lokale Verarmung und/oder Fehlstelle eines Verbindungselements des Gate-Dielektrikums, insbesondere durch eine lokale Verarmung und/oder Fehlstelle von Sauerstoff, und/oder
- durch Tempern und/oder
- durch Kombination verschiedener dielektrischer Materialien, z. B. Siliziumdioxid/$Ta_2O_5$, $SiO_2$/$Al_2O_3$ bzw $SiO_2$/High-K-Dielektrikum etc.
- durch mechanischen Stress oder andere Oberflächenzustände zwischen dem Kanal und anderen Materialien, z. B. zwischen Si und $Si_{1-x}Ge_x$
- durch eine lokale Schädigung des Gate-Dielektrikums mit ionisierender Strahlung oder Elektronenstrahlung, und/oder

- durch Implantation von Fremdionen in das Gate-Dielektrikum, und/oder
- durch mechanische Druck- und/oder Zugspannung, und/oder
- durch Injektion heißer Ladungsträger aus dem Kanal in das Gate-Dielektrikum bei temporärer Überlastung des Feldeffekttransistors

in das Gate-Dielektrikum eingebracht sein.

Bei einer Stapelung verschiedener dielektrischer Materialien kann es auf Grund unterschiedlicher Gitterkonstanten sowie mechanischer Verspannungen eine Interdiffusion zwischen den beiden dielektrischen Schichten geben. Auch Störstellen können zwischen zwei verschiedenen dielektrischen Schichten ausgetauscht werden. Man bezeichnet diese und weitere Möglichkeiten der Einflussnahme auf das Dielektrikum mit dem Sammelbegriff "dielectric engineering".

Mechanische Verspannungen als Oberflächenzustände können beispielsweise durch einen Unterschied in den Gitterkonstanten zweier Materialien entstehen oder auch durch Verbiegung des Substrats bewusst eingebracht werden. Durch den mechanischen Stress können einige Fehlstellen erzeugt werden.

Vorteilhaft ist die Anlagerstelle höchstens 2 nm vom leitenden Kanal des Feldeffekttransistors entfernt, um den Durchgriff des Ladungsaustausches an der Anlagerstelle auf den Strom durch den Kanal zu verstärken. Dies steht im Widerspruch zu den üblichen Design-Zielen bei Feldeffekttransistoren, bei denen der Einfluss des Telegrafenrauschens an Defekten auf den Strom durch den Transistor minimiert werden soll.

In einer besonders vorteilhaften Ausgestaltung der Erfindung weist die Vorrichtung Mittel zur Auswertung der Zeitkonstanten $\tau_c$ für das Einfangen und/oder $\tau_e$ für das Abgeben von Ladungsträgern durch die Anlagerstelle aus statistischen stufenartigen Fluktuationen des Drain-Stroms (Telegrafen-Modulationssignal) auf, die von der Anlagerstelle ausgehen. Die einzige Unbekannte, von der $\tau_c$ abhängt, ist das zu messende Potential bzw. die zu messende Spannung. $\tau_e$ hängt dagegen nicht vom Potential bzw. von der Spannung ab, sondern im Wesentlichen nur von der Temperatur. Die Genauigkeit, mit der die Zeitkonstanten bestimmbar sind, steigt mit zunehmender Messzeit, indem sich die Statistik verbessert.

Für die statistische Auswertung der Zeitkonstanten ist es besonders vorteilhaft, wenn die Vorrichtung Mittel zur Auswertung der Häufigkeitsverteilung unterschiedlich langer Berge und/oder Täler im Zeitverlauf des Drain-Stroms aufweist. Es stehen dann nicht nur unterschiedliche Methoden zur letztendlichen Ermittlung der Zeitkonstanten zur Verfügung (etwa Mittelwert und Median), sondern die Form und Ausdehnung der Verteilung liefert zusätzlich eine Information über die Verlässlichkeit und Qualität des so ermittelten Wertes. Die Häufigkeitsverteilung ist idealerweise eine Lorentz-Verteilung.

Vorteilhaft weist die Vorrichtung Mittel zur Auswertung des Frequenzspektrums des Drain-Stroms auf. Aus diesem Frequenzspektrum lässt sich die Grenzfrequenz $f_0$ ermitteln, mit der das Telegrafen-Modulationssignal gerade noch übertragen wird. Diese Grenzfrequenz $f_0$ ist über

$$f_0 = \frac{\tau_c + \tau_e}{\tau_c \cdot \tau_e} = \frac{1}{\tau_c} + \frac{1}{\tau_e} \qquad (1)$$

mit den beiden Zeitkonstanten $\tau_c$ und $\tau_e$ verknüpft. Wenn $\tau_c \ll \tau_e$, dann kann in Gleichung (1) der Beitrag von $\tau_e$ zu $f_0$ vernachlässigt werden, und es gilt näherungsweise

$$f_0 \approx \frac{1}{\tau_c} \qquad (2)$$

$\tau_c$ ist in den meisten Anwendungen die interessierende Messgröße, die beispielsweise sehr stark vom pH-Wert der Probe abhängt.

Wenn $\tau_c$ nicht sehr klein gegen $\tau_e$ ist, muss der Beitrag von $\tau_e$ zu $f_0$ berücksichtigt werden. Dieser Beitrag hängt sowohl von der Temperatur als auch von der Gatespannung ab. Vorteilhaft wird die Messung daher bei einer Temperatur und/oder Gatespannung durchgeführt, bei der $\tau_c \ll \tau_e$ gilt, insbesondere $\tau_c < 0.1 * \tau_e$.

Für die näherungsweise Bestimmung der Grenzfrequenz $f_0$ genügt an Stelle eines kompletten Frequenzspektrums auch eine Messung des Zeitverlaufs, aus der anschließend Frequenzkomponenten extrahiert werden. Da $\tau_e$ näherungsweise nur von der Temperatur abhängt und ansonsten konstant ist, kann bei hinreichend konstanter Temperatur eine Messung von $f_0$ bei einigen wenigen verschiedenen Biasspannungen am Gate bereits ausreichen, um $\tau_c$ und damit das gesuchte Potential bzw. die gesuchte Spannung zu erhalten.

Die Anzahl der Anlagerstellen, die durch Kontakt der Messvorrichtung mit der Probe gleichzeitig beeinflusst werden, lässt sich steuern, indem nach Möglichkeit nur eine oder nur einige wenige solcher Anlagerstellen in das Gate-Dielektrikum eingebracht werden. Beispielsweise können hierfür die räumlichen Abmessungen des Feldeffekttransistors so weit verkleinert werden, dass sich im Gate-Dielektrikum mit hoher Wahrscheinlichkeit mindestens eine, aber auch nicht mehr als einige wenige Anlagerstellen befinden. Jede Anlagerstelle ruft quasi ihre charakteristischen Zeitkonstanten laut aus. Sind mehrere Stimmen zu hören, kann zunächst mehr derartige Information gleichzeitig übertragen werden; ab einer kritischen Anzahl von Stimmen sind diese dagegen nicht mehr voneinander zu unterscheiden, und es wird gar keine Information mehr übertragen.

Alternativ oder in Kombination zur Verkleinerung des Transistors oder zur Kontrolle der Anlagerstellen bei dessen Herstellung lässt sich deren Anzahl einschränken, indem die Probe auf einer Fläche mit dem Gate des Feldeffekttransistors in Kontakt kommt, die in mindestens einer kartesischen Raumdimension eine Abmessung zwischen 20 und 500 nm, bevorzugt zwischen 20 und 100 nm, hat. In diesem Bereich wird mit hoher Wahrscheinlichkeit mindestens eine Anlagerstelle durch die Probe beeinflusst, so dass die Vorrichtung ein Signal liefern kann. Es werden aber auch mit hoher Wahrscheinlichkeit nicht mehr als einige wenige Anlagerstellen beeinflusst, so dass Signalbeiträge von verschiedenen Anlagerstellen noch voneinander unterscheidbar sind. Besonders bevorzugt hat die Fläche in beiden kartesischen Raumdimensionen eine Abmessung zwischen 20 und 500 nm, ganz besonders bevorzugt zwischen 20 und 100 nm.

Die Fläche, auf der die Probe mit dem Gate in Kontakt kommt, lässt sich beispielsweise bei einer flüssigen Probe dadurch eingrenzen, dass das Gate bedeckt ist und nur die definierte Fläche für den Zutritt der Probe freigelegt ist. Bei einer festen Probe lässt sich die Fläche eingrenzen, indem eine entsprechend kleine Probe strukturiert wird.

Mit der erfindungsgemäßen Vorrichtung lassen sich beispielsweise in einer Flüssigkeit einzelne Moleküle, etwa DNA, nachweisen. Da eine verlässliche Messung nur wenige Mikrosekunden dauert, können aber auch dynamische Phänomene in Echtzeit studiert werden, etwa die Faltung von Proteinen, die Katalyse von Reaktionen durch Enzyme oder die Hybridisierung von DNA.

Für die Messung des Stroms durch den Transistor ist der in der deutschen Patentanmeldung 10 2010 021 078.1 beschriebene und beanspruchte rauscharme Verstärker besonders geeignet.

Sämtliche für die Vorrichtung gegebene Offenbarung gilt ausdrücklich auch für das im Folgenden offenbarte Verfahren. Umgekehrt gilt sämtliche für das Verfahren gegebene Offenbarung ausdrücklich auch für die Vorrichtung.

Im Rahmen der Erfindung wurde ein Verfahren zur Messung kleiner Spannungen und Potentiale oder anderer elektrischer Größen einer Probe entwickelt. Das Verfahren wird durch den unabhängigen Anspruch 9 definiert.

Es wurde erkannt, dass bei der bislang üblichen Zeitmittelung des durch den Transistor getriebenen Stroms ein erheblicher Anteil der im Signal enthaltenen Information verworfen wurde. Der Zeitverlauf des Signals enthält sowohl Berge als auch Täler. Aus beiden kann eine Information über das gesuchte Potential bzw. die gesuchte Spannung gewonnen werden. Wird nun eine Mittelung über den gesamten Zeitverlauf durchgeführt, löschen sich viele der Berge und Täler gegenseitig aus, was bislang auch erwünscht war. Die Erfinder haben erstmals erkannt, dass die Berge und Täler im Gegensatz zur bisherigen Praxis nicht unnütz sind. Jeder Berg und jedes Tal ist ein Stück Statistik, das zur Bestimmung der Zeitkonstanten $\tau_c$ und $\tau_e$ beiträgt und somit deren Genauigkeit verbessert. Damit ist auch das gesuchte Potential bzw. die gesuchte Spannung, von der $\tau_c$ abhängt, genauer bestimmbar. Bei gegebener Messzeit wird also eine viel größere Menge an Information genutzt. Umgekehrt kann eine vorgegebene Genauigkeit in kürzerer Messzeit erzielt werden.

Für diese Auswertung wird vorteilhaft mindestens eine Zeitkonstante aus der Häufigkeitsverteilung unterschiedlich langer Berge und/oder Täler im Drain-Strom ausgewertet. Alternativ oder auch in Kombination hierzu wird mindestens eine Zeitkonstante aus dem Frequenzspektrum des Drain-Stroms ausgewertet.

Das gemessene Potential bzw. die gemessene Spannung ist in aller Regel kein Selbstzweck, sondern ein Maß für eine chemische oder biologische Eigenschaft der Probe, die sich in einer Aufnahme, Abgabe oder räumlichen Umorganisation von Ladungen äußert. Indem das Potential bzw. die Spannung erfindungsgemäß schneller und genauer bestimmt werden kann, wird auch die Messung der letztendlich gesuchten Größe verbessert.

In einer besonders vorteilhaften Ausgestaltung der Erfindung wird aus der Zeitkonstante $\tau_c$ für das Einfangen von Ladungsträgern durch die Anlagerstelle oder aus der Grenzfrequenz $f_0$ aus dem Frequenzspektrum des Drain-Stroms der pH-Wert der Probe ausgewertet. Der pH-Wert ist direkt damit korreliert, wie viele Wasserstoffionen sich am Gate des Feldeffekttransistors anlagern und dort zum lokalen Potential beitragen. Damit ist er mit diesem Potential unmittelbar korreliert und kann beispielsweise aus Messwerten für das Potential ausgewertet werden. Somit handelt es sich beim pH-Wert um eine elektrische Größe der Probe. Wenn der pH-Wert die einzige im konkreten Anwendungsfall interessierende Eigenschaft der Probe ist, kann auch an Hand von Testproben mit definiertem pH-Wert eine direkte Eichung vorgenommen werden, die jeder Zeitkonstanten $\tau_c$ bzw. jeder Grenzfrequenz $f_0$ genau einen pH-Wert zuordnet. Auf der Basis einer solchen Eichung kann der pH-Wert ohne Umweg über eine Spannung oder ein Potential direkt aus $\tau_c$ bzw. aus $f_0$ ausgewertet werden.

Spezieller Beschreibungsteil

Nachstehend wird der Gegenstand der Erfindung an Hand von Figuren erläutert, ohne dass der Gegenstand der Erfindung hierdurch beschränkt wird. Es ist gezeigt:

Figur 1: Ausführungsbeispiel der erfindungsgemäßen Vorrichtung in Schnittzeichnung (Teilbild a) und Zeitverlauf des durch den Kanal fließenden Stroms (Teilbild b).

Figur 2: Rasterelektronenmikroskopische Aufnahme eines Nanodraht-Feldeffekttransistors als Ausführungsbeispiel der erfindungsgemäßen Vorrichtung.

Figur 3: Auswirkung des pH-Werts der Probe auf die funktionale Abhängigkeit des Drain-Stroms

$I_D$ von der Gate-Spannung $V_G$.

Figur 4: Auswirkung des pH-Werts der Probe auf die Grenzfrequenz $f_0$ des Telegrafen-Modulationssignals.

Figur 5: Vergleich der Empfindlichkeit von pH-Messungen über die Zeitkonstante $\tau_c$ des Telegrafen-Modulationssignals (obere Kurve) und über den Drain-Strom $I_D$ (untere Kurve).

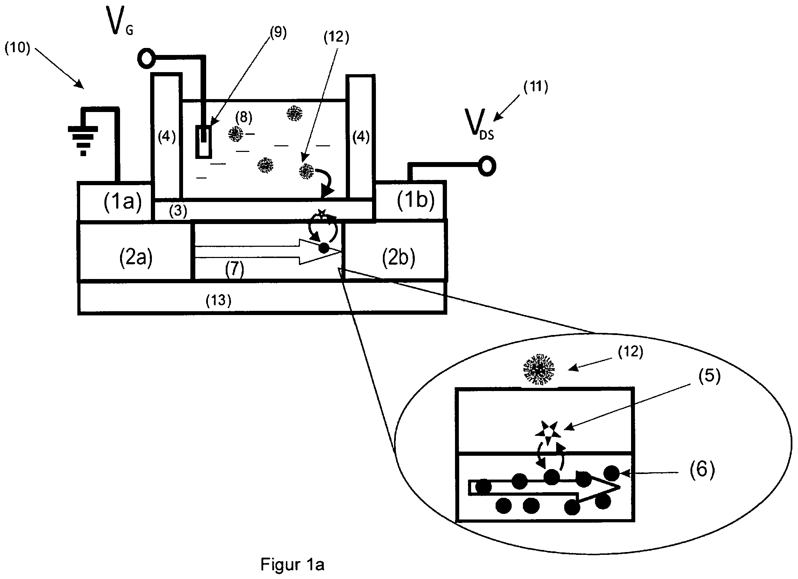

Figur 1a zeigt ein Ausführungsbeispiel der erfindungsgemäßen Vorrichtung in Schnittzeichnung. Auf einem Substrat 13 befinden sich dotierte, hochimplantierte halbleitende Bereiche 2a und 2b, die über Metallkontakte 1a und 1b mit der Außenwelt kontaktiert sind. Zwischen den Bereichen 2a und 2b befindet sich ein halbleitender Nanokanal 7, der deutlich weniger stark dotiert ist als die Bereiche 2a und 2b. Der Kontakt 1a ist mit einer Erdung 10 und der Kontakt 1b mit einer Spannungsquelle 11 für die Drain-Source-Spannung $V_{DS}$ verbunden. In dieser Schaltung funktioniert der Bereich 2a als Source, und der Bereich 2b funktioniert als Drain. Die durch den Nanokanal 7 transportierten und dort als ausgefüllte Kreise dargestellten Ladungsträger 6 sind Löcher. Ihre Fließrichtung ist durch den im Nanokanal 7 eingezeichneten Pfeil angegeben.

Der Nanokanal 7 und Teile der Bereiche 2a und 2b sind mit dem Gate-Dielektrikum 3 abgedeckt. Das Gate-Dielektrikum 3 bildet zugleich den elektrisch isolierenden Boden eines Reservoirs 4 für die Probenflüssigkeit 8. Das Reservoir 4 isoliert die Probenflüssigkeit 8 gegen die Kontakte 1a und 1b. Das Potential der Probenflüssigkeit 8 kann über eine Referenzelektrode 9 mit einer Spannung $V_G$ gesetzt werden. Die Probenflüssigkeit 8 enthält die zu studierenden Moleküle 12.

Das Gate-Dielektrikum 3 enthält in seinem Inneren, in der Nähe der Grenzfläche zum Nanokanal 7, einen Punktdefekt 5, der einen Ladungsträger 6 (ein Loch) mit dem Nanokanal 7 auszutauschen vermag. Zu jedem Zeitpunkt ist der Punktdefekt 5 entweder mit einem Loch 6 belegt oder nicht. Dies wird in der Ausschnittsvergrößerung verdeutlicht.

Der Punktdefekt ist negativ geladen, wenn er nicht mit einem Ladungsträger 6 (einem Loch) belegt ist. Es bildet sich im Kanal in der Nähe des Defekts eine lokale Verarmungszone, in der sich Löcher anlagern und für den Stromtransport durch den Kanal nicht mehr zur Verfügung stehen.

Wenn der Punktdefekt 5 mit einem Ladungsträger 6 (einem Loch) belegt ist, wird er ladungsmäßig neutralisiert. In diesem Fall verschwindet die lokale Verarmungszone von Ladungsträgern in der Nähe des Punktdefekts, und der Stromtransport durch den Kanal wird nicht mehr behindert. Daher fließt ein höherer Strom durch den Nanokanal als wenn der Punktdefekt nicht belegt ist. Indem der Punktdefekt 5 in statistischer Zeitfolge zwischen den beiden Zuständen "belegt" und "unbelegt" wechselt, werden statistische, stufenartige Fluktuationen zwischen zwei Niveaus auf den durch den Nanokanal 7 fließenden Strom aufmoduliert.

Lagert sich eines der zu studierenden Moleküle 12 an der Oberfläche des Gate-Dielektrikums 3 an, so hat dies zwei Wirkungen:

- Der durch den Nanokanal 7 fließende Strom wird auf Grund der elektrostatischen Beeinflussung durch das Molekül 12 um einen konstanten Offset $\Delta I^b$ geändert.
- Es ändern sich auch die Zeitkonstanten $\tau_c$ für das Einfangen eines Lochs 6 durch den Punktdefekt 5 (sehr stark) und $\tau_e$ für die Rückgabe dieses Lochs 6 an den Nanokanal 7 (deutlich weniger stark). Über den Unterschied in den Zeitkonstanten kann beispielsweise das durch das Probenmolekül 12 geänderte lokale Potential am Gate, die Konzentration der Moleküle 12 in der Probenflüssigkeit 8 oder der pH-Wert der Probenflüssigkeit 8 bestimmt werden.

Das Substrat 13 kann beispielsweise ein undotierter Halbleiter, ein mit einer Oxidschicht passivierter Halbleiter (etwa ein SOI-Substrat) oder ein dielektrisches Substrat sein.

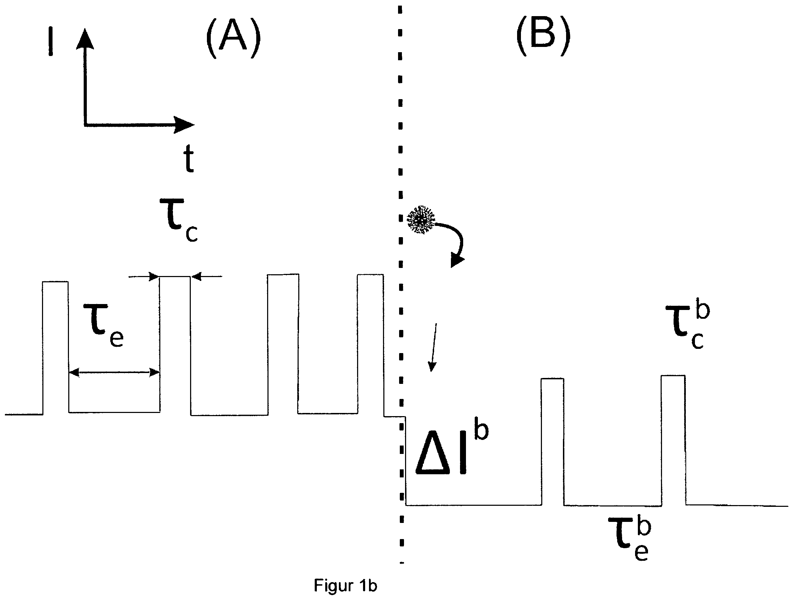

Figur 1b zeigt den Zeitverlauf des durch den Kanal fließenden Stroms ohne (Bereich A) und mit (Bereich B) an der Oberfläche des Gate-Dielektrikums 3 angelagertes Probenmolekül 12. Der Strom fluktuiert statistisch zwischen zwei Niveaus, die den beiden Zuständen "Punktdefekt 5 ist belegt" und "Punktdefekt 5 ist frei" entsprechen.

Dabei liegt der Zustand höheren Stroms vor, wenn der Punktdefekt 5 mit einem Loch belegt ist. Die mittlere Dauer, für die dieser Zustand anhält, ist die Zeitkonstante $\tau_c$. Die Dauern der einzelnen Phasen, in denen dieser Zustand vorliegt, streuen mit einer Lorentz-Verteilung um den Mittelwert $\tau_c$.

Der Zustand niedrigeren Stroms liegt vor, wenn der Punktdefekt 5 frei ist. Die mittlere Dauer, für die dieser Zustand anhält, ist die Zeitkonstante $\tau_e$. Die Dauern der einzelnen Phasen, in denen dieser Zustand vorliegt, streuen mit einer Lorentz-Verteilung um den Mittelwert $\tau_e$.

Nach der Bindung des Probenmoleküls 12 sind sowohl die Verschiebung des Stroms um den Offset $\Delta I^b$ als auch die Änderung in den Zeitkonstanten $\tau_c^b$ und $\tau_e^b$ deutlich sichtbar.

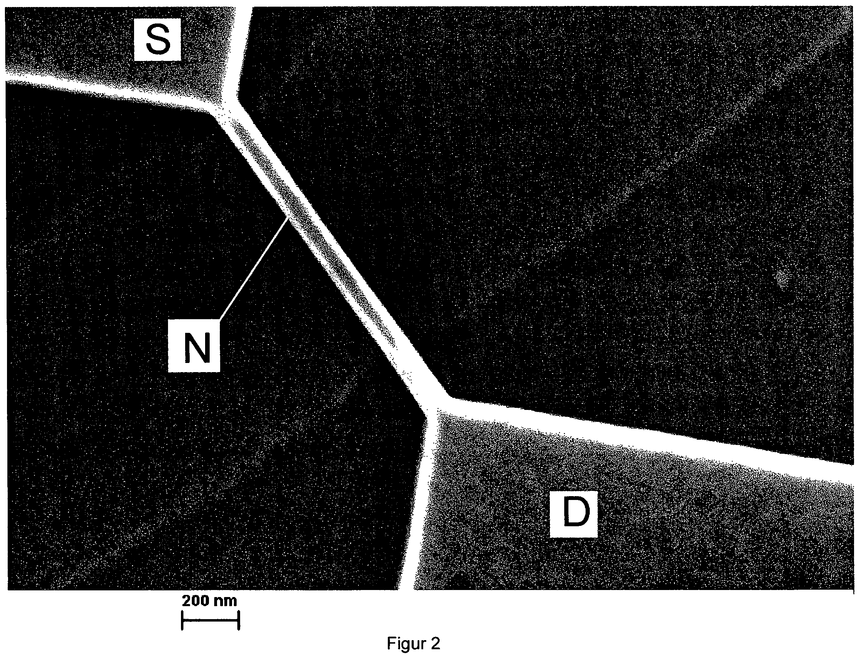

Figur 2 zeigt ein Ausführungsbeispiel der erfindungsgemäßen Vorrichtung. Die Figur ist eine rasterelektronenmikroskopische Aufnahme des $p^+$-p-$p^+$ Silizium-Nanodraht-Feldeffekttransistors. Der von Source S nach Drain D verlaufende Nanodraht N ist 500 nm lang, 100 nm breit und 50 nm dick. Er wird typischerweise mit einer Drain-Source-Spannung von -0.1 V und einer Gate-Biasspannung von -0.9 V betrieben. Der Transistor war bei den Messungen durch eine $SiO_2$-Passivierungsschicht von 9 nm Dicke vor der aggressiven Probe geschützt. Bei einer weiteren Verkleinerung der charakteristischen Längen des Transistors auf etwa 20 nm erwarten die Erfinder eine weitere Steigerung der Empfindlichkeit um mehrere Größenordnungen. Für die Herstellung nanoskaliger Transistoren haben sich in den Versuchen der Erfinder Nanoimprint-Verfahren als besonders vorteilhaft erwiesen.

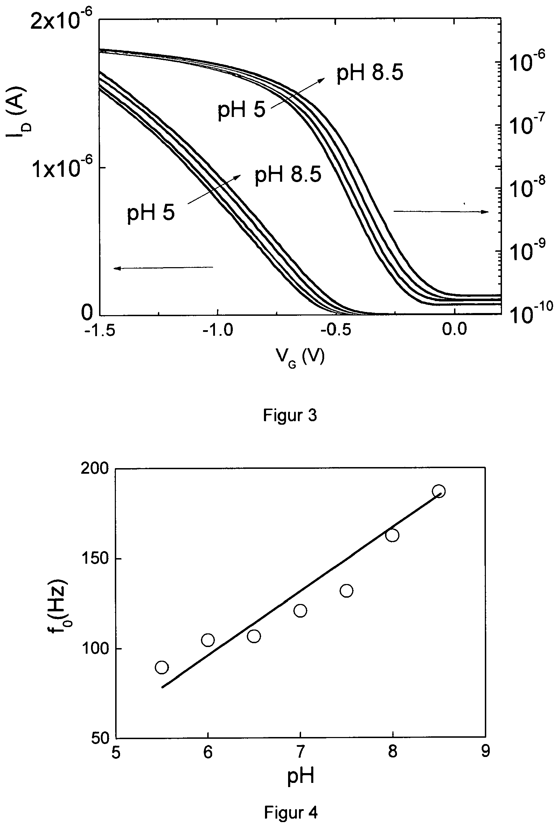

Figur 3 zeigt Verläufe des Drain-Stroms $I_D$ in Abhängigkeit der über die Referenzelektrode 9 vorgelegten Gate-Spannung $V_G$ für verschiedene pH-Werte der Probenflüssigkeit 8. Für die linke Kurvenschar ist der Strom linear aufgetragen (linke Skala), für die rechte Kurvenschar ist der Strom logarithmisch aufgetragen (rechte Skala). Nach dem Stand der Technik wurde der pH-Wert aus diesen Stromverläufen ausgewertet. Obwohl sich die pH-Werte über einen breiten Bereich zwischen 5.5 und 8.5 erstrecken, ändern sich die Stromverläufe nur schwach. Die Messmethode gemäß Stand der Technik ist daher vergleichsweise unempfindlich.

Figur 4 zeigt ein Ausführungsbeispiel des erfindungsgemäßen Verfahrens, durchgeführt mit der erfindungsgemäßen Vorrichtung. Für die gleichen pH-Werte der Probenflüssigkeit wie in Figur 3 ist jeweils die aus dem Frequenzspektrum des Drain-Stroms ermittelte Grenzfrequenz $f_0$ als Funktion des pH-Werts aufgetragen. Es wurde zum ersten Mal gezeigt, dass eine Änderung des pH-Wertes die Grenzfrequenz $f_0$ deutlich stärker ändert als den in Figur 3 gezeigten Stromverlauf.

Diese Änderung kann noch deutlicher herausgearbeitet werden, indem das Frequenzspektrum des Drainstroms mehrfach, beispielsweise mindestens 10-fach, bevorzugt mindestens 50-fach und ganz besonders bevorzugt mindestens 100-fach, aufgenommen und die Einzelmessungen durch Mittelung zu einem Endergebnis für das Spektrum zusammengefasst werden. Da $\tau_e$ nicht vom pH-Wert der Probenflüssigkeit abhängt, ist sein Einfluss auf $f_0$ rein statistisch und schwindet umso mehr, je mehr Einzelmessungen in die Mittelung eingehen. In dem in Figur 4 gezeigten Beispiel liegt $\tau_e$ in der gleichen Größenordnung wie $\tau_c$.

Der mit der Mittelung einhergehenden Verlängerung der Messzeit kann entgegengewirkt werden, indem das Spektrum nur in demjenigen Frequenzbereich aufgenommen wird, in dem die Grenzfrequenz $f_0$ auf Grund einer Änderung des pH-Werts der Probenflüssigkeit variieren kann.

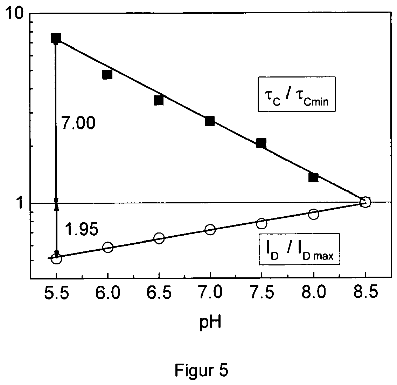

Figur 5 vergleicht in einem weiteren Ausführungsbeispiel die Empfindlichkeit des erfindungsgemäßen Verfahrens zur Messung des pH-Werts einer flüssigen Probe (Kurve A, Messpunkte durch ausgefüllte Rechtecke symbolisiert) mit der Empfindlichkeit eines Verfahrens gemäß Stand der Technik, bei dem nur der Zeitmittelwert des Drain-Stroms ausgewertet wurde (Kurve B, Messpunkte durch nicht ausgefüllte Kreise symbolisiert).

Der pH-Wert der Probe wurde ausgehend von 5.5 in Schritten von 0.5 bis auf 8.5 gefahren. Bei dem erfindungsgemäßen Verfahren wurde jeweils die Zeitkonstante $\tau_c$ für den Einfang von Ladungsträgern 6 aus dem Kanal 7 in den Punktdefekt 5 gemessen. Bei dem Verfahren gemäß Stand der Technik wurde jeweils der Zeitmittelwert $I_D$ des Drain-Stroms gemessen, analog zu Figur 3. Der Unterschied im Signal zwischen dem minimalen und dem maximalen pH-Wert beträgt beim Verfahren gemäß Stand der Technik 1.95, beim erfindungsgemäßen Verfahren dagegen 7. Die Empfindlich-

**Patentansprüche**

1. Vorrichtung zur Messung kleiner Spannungen und Potentiale oder anderer elektrischer Größen einer Probe, umfassend

   - mindestens einen Feldeffekttransistor mit Source (2a), Drain (2b) Gate sowie ein Gate-Dielektrikum (3),
   - wobei das Gate durch das Gate-Dielektrikum (3) vom leitenden Kanal (7) des Feldeffekttransistors isoliert ist, und
   - wobei das Gate-Dielektrikum (3) in seinem Inneren mindestens eine Anlagerstelle (5) aufweist, die Ladungsträger (6) aus dem Kanal (7) einzufangen und an den Kanal abzugeben vermag,
   - Mittel (11) zum Anlegen einer Spannung zwischen Source und Drain und
   - Mittel (9) zur Beaufschlagung des Gates mit einer Biasspannung, wobei das Gate-Dielektrikum mit der Probe in Kontakt steht.

2. Vorrichtung nach Anspruch 1, **dadurch gekennzeichnet, dass** die Anlagerstelle ein lokaler Defekt im Gate-Dielektrikum ist.

3. Vorrichtung nach einem der vorherigen Ansprüche, **dadurch gekennzeichnet, dass** das Gate-Dielektrikum (3) den elektrisch isolierenden Boden eines Reservoirs (4) für eine Probenflüssigkeit (8) bildet, die die zu studierenden Moleküle (12) enthält.

4. Vorrichtung nach einem der Ansprüche 1 bis 3, **dadurch gekennzeichnet, dass** die Anlagerstelle höchstens 2 nm vom leitenden Kanal des Feldeffekttransistors entfernt ist.

5. Vorrichtung nach einem der Ansprüche 1 bis 4, **dadurch gekennzeichnet, dass** sie Mittel zur Auswertung der Zeitkonstanten für das Einfangen und/oder für das Abgeben von Ladungsträgern durch die Anlagerstelle aus statistischen stufenartigen Fluktuationen des Drain-Stroms aufweist, die von der Anlagerstelle ausgehen.

6. Vorrichtung nach einem der Ansprüche 1 bis 5, **dadurch gekennzeichnet, dass** sie Mittel zur Auswertung der Häufigkeitsverteilung unterschiedlich langer Berge und/oder Täler im Zeitverlauf des Drain-Stroms aufweist.

**7.** Vorrichtung nach einem der Ansprüche 1 bis 6, **dadurch gekennzeichnet, dass** sie Mittel zur Auswertung des Frequenzspektrums des Drain-Stroms aufweist.

**8.** Vorrichtung nach einem der Ansprüche 1 bis 7, **dadurch gekennzeichnet, dass** die Probe auf einer Fläche mit dem Gate des Feldeffekttransistors in Kontakt steht, die in mindestens einer kartesischen Raumdimension eine Abmessung zwischen 20 und 500 nm, bevorzugt zwischen 20 und 100 nm, hat.

**9.** Verfahren zur Messung kleiner Spannungen und Potentiale oder anderer elektrischer Größen einer Probe, wobei die Probe mit dem Gate-Dielektrikum eines Feldeffekttransistors in Kontakt steht, zwischen dessen Source und Drain eine Spannung anliegt, und statistische stufenartige Fluktuationen im zwischen Drain und Source getriebenen Strom ausgewertet werden, die von einer zwischen Probe und leitendem Kanal des Feldeffekttransistors angeordneten Anlagerstelle innerhalb des Gate-Dielektrikums ausgehen, welche Ladungsträger aus dem Kanal einfängt und an den Kanal abgibt, und wobei die Spannung bzw. das Potential aus der oder den Zeitkonstanten für das Einfangen und/oder für das Abgeben von Ladungsträgern durch die Anlagerstelle ausgewertet wird.

**10.** Verfahren nach Anspruch 9, **dadurch gekennzeichnet, dass** mindestens eine Zeitkonstante aus der Häufigkeitsverteilung unterschiedlich langer Berge und/oder Täler im Drain-Strom ausgewertet wird.

**11.** Verfahren nach einem der Ansprüche 9 bis 10, **dadurch gekennzeichnet, dass** mindestens eine Zeitkonstante aus dem Frequenzspektrum des Drain-Stroms ausgewertet wird.

**12.** Verfahren nach einem der Ansprüche 9 bis 11, **dadurch gekennzeichnet, dass** aus der Zeitkonstante $\tau_c$ für das Einfangen von Ladungsträgern durch die Anlagerstelle oder aus der Grenzfrequenz $f_0$ aus dem Frequenzspektrum des Drain-Stroms der pH-Wert der Probe ausgewertet wird.

**Claims**

**1.** Device for measuring small voltages and potentials or other electrical variables of a sample, comprising

- at least one field effect transistor with source (2a), drain (2b) and gate as well as a gate dielectric (3),
- in which the gate is isolated from the conductive channel (7) of the field effect transistor through the gate dielectric (3) and
- in which the gate dielectric (3) has at least one accumulation point (5) inside it, which is able to capture the charge carriers (6) from the channel (7) and deliver them to the channel,
- means (11) for applying a voltage between the source and the drain and
- means (9) for applying a bias voltage to the gate,

in which
the gate dielectric is in contact with the sample.

**2.** Device according to claim 1, **characterised in that** the accumulation point is a local defect in the gate dielectric.

**3.** Device according to one of the previous claims, **characterised in that**
the gate dielectric (3) forms the electrically isolating floor of a reservoir (4) for a sample liquid (8), which contains the molecules to be studied (12).

**4.** Device according to one of claims 1 to 3, **characterised in that** the accumulation point is at most 2 nm away from the conductive channel of the field effect transistor.

**5.** Device according to one of claims 1 to 4, **characterised in that** it has means for evaluating the time constants for capturing and/or for delivering charge carriers through the accumulation point from statistically step type fluctuations of the drain current, which come from the accumulation point.

**6.** Device according to one of claims 1 to 5, **characterised in that** it has means for evaluating the frequency distribution of peaks and/or troughs of differing length in the time behaviour of the drain current.

**7.** Device according to one of claims 1 to 6, **characterised in that** it has means for evaluating the frequency spectrum of the drain current.

**8.** Device according to one of claims 1 to 7, **characterised in that** the sample is in contact with the gate of the field effect transistor on a surface, which has a dimension of between 20 and 500 nm, preferably between 20 and 100 nm, in at least one Cartesian spatial dimension.

**9.** Method for measuring small voltages and potentials or other electrical variables of a sample, in which the sample is in contact with the gate dielectric of a field effect transistor, a voltage is applied between its source and drain and statistically step type fluctuations in the current between the drain and the source are evaluated, which come from an accumulation point arranged between the sample and the conductive channel of the field effect transistor inside the gate dielectric, which captures charge carriers from the channel and delivers them to the channel, and in which the voltage or the potential is evaluated from the time constant or constants for capturing and/or delivering charge carriers through the accumulation point.

10. Method according to claim 9, **characterised in that** at least one time constant is evaluated from the frequency distribution of peaks and/or troughs of differing length in the drain current.

11. Method according to one of claims 9 to 10, **characterised in that** at least one time constant is evaluated from the frequency spectrum of the drain current.

12. Method according to one of claims 9 to 11, **characterised in that** the pH value of the sample is evaluated from the time constants $\tau_c$ for capturing charge carriers through the accumulation point or from the limit frequency $f_0$ from the frequency spectrum of the drain current.

## Revendications

1. Dispositif de mesure de tensions et potentiels électriques bas ou d'autres grandeurs électriques d'un échantillon, comprenant

   - au moins un transistor à effet de champ ayant une source (2a), un drain (2b), une grille ainsi qu'un diélectrique (3) de grille,
   - la grille étant isolée du canal (7) conducteur du transistor à effet de champ par le diélectrique (3) de grille, et
   - dans lequel le diélectrique (3) de grille a, à l'intérieur, au moins un point (5) de piégeage, qui peut capturer les porteurs (6) de charge sortant du canal (7) et les céder au canal,
   - des moyens (11) pour appliquer une tension entre la source et le drain et
   - des moyens (9) pour soumettre la grille à une tension de polarisation,

   dans lequel le diélectrique de grille est en contact avec l'échantillon.

2. Dispositif suivant la revendication 1, **caractérisé en ce que** le point de piégeage est un défaut local du diélectrique de grille.

3. Dispositif suivant l'une des revendications précédentes, **caractérisé en ce que** le diélectrique (3) de grille forme le fond isolant électriquement d'un réservoir (4) pour un liquide (8) échantillon, qui contient les molécules (12) à étudier.

4. Dispositif suivant l'une des revendications 1 à 3, **caractérisé en ce que** le point de piégeage est éloigné au plus de 2 nm du canal conducteur du transistor à effet de champ.

5. Dispositif suivant l'une des revendications 1 à 4, **caractérisé en ce qu'**il a des moyens d'exploitation des constantes de temps pour le piégeage et/ou la cession de porteurs de charge par le point de piégeage à partir de fluctuations statistiques en paliers du courant de drain, qui proviennent du point de piégeage.

6. Dispositif suivant l'une des revendications 1 à 5, **caractérisé en ce qu'**il a des moyens d'exploitation de la répartition de fréquence de crêtes et/ou de creux de longueur différente au cours du temps du courant de drain.

7. Dispositif suivant l'une des revendications 1 à 6, **caractérisé en ce qu'**il a des moyens d'exploitation du spectre de fréquence du courant de drain.

8. Dispositif suivant l'une des revendications 1 à 7, **caractérisé en ce que** l'échantillon est en contact sur une surface avec la grille du transistor à effet de champ, qui a, dans au moins une dimension cartésienne de l'espace, une dimension comprise entre 20 et 500 nm, de préférence entre 20 et 100 nm.

9. Procédé de mesure de tensions et de potentiels électriques bas ou d'autres grandeurs électriques d'un échantillon, l'échantillon étant en contact avec le diélectrique de grille d'un transistor à effet de champ, entre la source et le drain duquel s'applique une tension et on évalue des fluctuations statistiques en échelons du courant entre le drain et la source, qui proviennent, d'un point de piégeage au sein du diélectrique de grille disposé entre l'échantillon et un canal conducteur du transistor à effet de champ, point de piégeage qui capture des porteurs de charge sortant du canal et les cède au canal, la tension ou le potentiel étant évalué à partir de la ou des constantes de temps de la capture et/ou de la cession de porteurs de charge par le point de piégeage.

10. Procédé suivant la revendication 9, **caractérisé en ce que** l'on évalue au moins une constante de temps à partir de la répartition de fréquence de creux et/ou de crêtes de longueur différente du courant de drain.

11. Procédé suivant l'une des revendications 9 à 10, **ca-**

**ractérisé en ce que** l'on évalue au moins une constante de temps à partir du spectre de fréquence du courant de drain.

12. Procédé suivant l'une des revendications 9 à 11, **caractérisé en ce que** l'on évalue le pH de l'échantillon à partir de la constante $\tau_c$ de temps du piégeage de porteurs de charge par le point de piégeage ou à partir de la fréquence $f_0$ limite dans le spectre de fréquence du courant de drain.

Figur 1a

EP 3 066 459 B1

Figur 1b

EP 3 066 459 B1

Figur 2

Figur 3

Figur 4

Figur 5



**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006231754 A1

- DE 102010021078

**In der Beschreibung aufgeführte Nicht-Patentliteratur**

- **S. SORGENFREI ; C. CHIU ; M. JOHNSTON ; C. NUCKOLLS ; K. L. SHEPARD.** Debye Screening in Single-Molecule Carbon Nanotube Field-Effect Sensors. *Nano Letters 2011,* 2011, vol. 11, 3739-3743
- **FEI LIU et al.** Determination of the Small Band Gap of Garbon Nanotubes Using the Ambipolar Random Telegraph Signal. *NANO LETTERS,* 01. Juli 2005, vol. 5 (7), 1333-1336

- Giant random telegraph signals in the carbon nanotubes as a single defect probe. **FEI LIU et al.** APPLIED PHYSICS LETTERS. AMERICAN INSTITUTE OF PHYSICS, 11. April 2005, vol. 86, 163102-163102
- Effects of defects near source or drain contacts of carbon nanotube transistors. **NENG-PING WANG et al.** EUROPHYSICS LEITERS: A LEDERS JOURNAL EXPLORING THE FRONTIERS OF PHYSICS. INSTITUTE OF PHYSICS PUBLISHING, 03. Dezember 2012, vol. 100, 47009